# EUROPEAN PATENT SPECIFICATION

(11) **EP 2 998 358 B1**
(45) Date of publication and mention of the grant of the patent: **13.02.2019**
(21) Application number: 13884717.3
(22) Date of filing: 16.05.2013
(51) Int. Cl.: A61L 31/12, A61L 31/14, C08K 3/32, C08L 101/16

(54) **BIODEGRADABLE MATERIAL**
BIOLOGISCH ABBAUBARES MATERIAL
MATIÈRE BIODÉGRADABLE

(43) Date of publication of application: 23.03.2016
(73) Proprietor: SofSera Corporation, Tokyo 160-0022 (JP)
(72) Inventor: KOGAI, Yasumichi, Tokyo 160-0022 (JP); KAWABE, Karl Kazushige, Tokyo 160-0022 (JP); YAMADA, Kenji, Tokyo 160-0022 (JP)
(74) Representative: Hoffmann Eitle
(86) International application number: PCT/JP2013/063729
(87) International publication number: WO 2014/184939

(56) References cited:
- EP-A1- 1 121 943
- WO-A1-2009/073680
- WO-A2-2009/009520
- WO-A2-2009/129316
- JP-A- 2001 192 337
- JP-A- 2008 143 957
- JP-A- 2008 156 213
- JP-A- 2009 538 688
- JP-A- 2011 184 615
- JP-A- 2011 506 619
- JP-A- 2011 506 641
- JP-A- 2012 523 853
- JP-A- 2013 059 550
- US-A1- 2007 278 720
- US-A1- 2011 118 827
- DATABASE WPI Week 200867 Thomson Scientific, London, GB; AN 2008-L39346 XP002765233, & JP 2008 156213 A (JAPAN SCI & TECHNOLOGY AGENCY) 10 July 2008 (2008-07-10)
- DATABASE WPI Week 200852 Thomson Scientific, London, GB; AN 2008-J12632 XP002765234, & JP 2008 143957 A (DOKURITSU GYOSEI HOJIN SANGYO GIJUTSU SO) 26 June 2008 (2008-06-26)
- DATABASE WPI Week 201164 Thomson Scientific, London, GB; AN 2011-M08862 XP002765235, & JP 2011 184615 A (GUNZE KK) 22 September 2011 (2011-09-22)
- DATABASE WPI Week 201326 Thomson Scientific, London, GB; AN 2013-F15064 XP002765236, & JP 2013 059550 A (GUNZE KK) 4 April 2013 (2013-04-04)

## Description

### Technical Field

The present invention relates to a biodegradable material.

### Background Art

As desirable materials for being used in vivo, for example, a stent or a bone fixed material, a biomaterial using a biodegradable polymer as a base material is being studied.

For example, Patent Literature 1 discloses a stent for a blood-vessel, which is formed by a polylactic acid (PLLA) that has the optical purity of L-body in a predetermined range and the weight average molecular weight in a predetermined range, is planted in vivo, and then, is degraded and disappeared in vivo.

However, there is a problem in that in the case of applying such biodegradable polyester in vivo, when the polyester is degraded in the body, acidic degradation products may be produced, thereby causing inflammation.

For such a problem, Patent Literature 2 discloses a stent that can allow acidic biodegradable polymer-degradation products to be neutralized by including calcium phosphate (for example, hydroxyapatite, and the like) treated with an alkali inorganic material such as calcium hydroxide, along with a polylactic acid that is a degradable polymer.

Patent Litterature 3 discloses a biomaterial comprising calcium phosphate particles selected from hydroxyapatite, tricalcium phosphate or calcium secondary phosphate having an average size of 0.1 to 200 microns and a copolymer of lactic acid, glycolic acid and epsilon-caprolactone. The preferred calcium phosphate is tricalcium phosphate.

### Citation List

### Patent Literature

Patent Literature 1: JP 2011-031064 A
Patent Literature 2: JP 2007-313009 A
Patent Litterature 3: EP 1 121 943

However, the stent disclosed in Patent Literature 2 has the mechanical strength that is not necessarily sufficient, and also, the insufficient effect in neutralizing the acidic component of the polyester-degradation products in some cases, thereby being the cases where the inflammation in vivo is not sufficiently suppressed.

### Summary of Invention

### Technical Problem

Accordingly, an object of the present invention is to provide a biodegradable material, which has the sufficient mechanical strength, considering in vivo use also, and can suppress pH change caused by the acidic degradation products of a biodegradable polymer.

### Solution to Problem

The present inventors conducted intensive studies, and as a result, found that the biodegradable material according to the claims can suppress the diffusion of acidic components caused by degrading the polymer over a long period and has sufficient mechanical strength.

Accordingly, the present inventors completed the present invention. In other words, the present invention has the following constitution.

According to the present invention, a biodegradable material is a biodegradable material including polyester and including hydroxyapatite particles having an average particle diameter of 10 to 500 nm. In addition, the hydroxyapatite particles are a sintered body. In addition, a content of the hydroxyapatite particles may be 0.1 to 50% by mass with respect to the total mass of the material.

In addition, the biodegradable material may be for an implantable biomaterial.

### Advantageous Effects of Invention

It is possible to provide a biodegradable material, which has the sufficient mechanical strength, considering in vivo use also, and can suppress pH change caused by the acidic degradation products of the biodegradable polymer.

### Brief Description of Drawings

Fig. 1 is a graph illustrating the changes with time of the weights of films according to Examples and Comparative Examples.
Fig. 2 is the SEM photographs of the surface morphologies of the films according to Examples and Comparative Examples.
Fig. 3 is a graph illustrating the results of measuring the weight average molecular weights of PLGA films prepared by immersing respective samples according to Examples and Comparative Examples in phosphate buffered normal saline (PBS), leaving the samples as it is at 37°C, and then being collected.
Fig. 4 is a graph illustrating the result of measuring pH of the solutions prepared by immersing respective samples according to Examples and Comparative Examples in normal saline and leaving the samples as it is at 37°C.

### Description of Embodiments

The biodegradable material according to the present embodiment is a material prepared by mixing the hydroxyapatite having a specific particle diameter with a biodegradable polyester base material. Hereinafter, the constitutional components, compositions, physical properties, preparing method, and use of the biodegradable material according to the present embodiment will be described.

### «Constitutional components of biodegradable material»

The constitutional components of the biodegradable material according to the present embodiment will be described. The biodegradable material according to the present embodiment is the material, in which biodegradable polyester is used as a base material, and the hydroxyapatite particles are included in the corresponding base material. Hereinafter, polyester (polyester base material) and calcium phosphate particles that are the raw materials of the biodegradable material according to the present embodiment will be described.

### <Polyester base material>

The biodegradable material according to the present embodiment uses polyester as a base material. When the corresponding polyester is used as biodegradable polyester, for example, in a case in which this material is applied in vivo, this material is degraded over the years, and finally is not remained in vivo (it is not remained in a body as a foreign material). Here, examples of the biodegradable polyester may include crystalline resins such as aliphatic polyester prepared by performing the polycondensation of aliphatic dicarboxylic acid and aliphatic diol as a main component, aliphatic polyester prepared by performing the ring-opening polymerization of cyclic lactones, synthetic aliphatic polyester, and aliphatic polyester biosynthesized in a bacterial cell. Examples of the aliphatic polyester resin may include polyoxalic acid ester, polysuccinic acid ester, polyhydroxybutyric acid, polydiglycolic acid butylene, polycaprolactone, polydioxanone, hydroxyl carboxylic acid-based aliphatic polyester resin such as oxyacid polymer such as lactic acid, malic acid, or glycolic acid, or copolymers thereof, and the like. Among them, in terms of formability, heat resistance, impact resistance, and biodegradability, hydroxyl carboxylic acid-based aliphatic polyester resin (especially, polylactic acid) is preferred.

### <Calcium phosphate particles>

Next, a type, a sintered body in a suitable form, a particle diameter, and a preparing method of calcium phosphate particle that is used for the biodegradable material according to the present embodiment will be described.

### <Type>

Calcium phosphate is a salt containing of a calcium ion and a phosphate ion, and in detail, examples thereof may include monocalcium phosphate, dicalcium phosphate, tricalcium phosphate (tricalcium α-phosphate and tricalcium β-phosphate), tetracalcium phosphate, octacalcium phosphate, calcium hydrogen phosphate, calcium dihydrogen phosphate, hydroxyapatite (HAp), fluoroapatite (FAp), apatite carbonate (CAp), silver apatite (AgHAp), and the like. Among them, hydroxyapatite particles are used as a calcium phosphate particle, and thus, it is possible to improve acid neutralizing capacity. In addition, the hydroxyapatite (HAp) described herein represents basic calcium phosphate represented by Chemical Formula Ca₁₀(PO₄)₆(OH)₂.

### <Sintered body>

As hydroxyapatite particles, hydroxyapatite particles that are sintered (hereinafter, referred to as sintered hydroxyapatite particles) are used. By sintering calcium phosphate particles (for example, at 800°C for 1 hour), the crystallinity of particles increases, and also the aggregate of a plurality of primary particles is fused by heat, thereby making the particles more strong and stable. In addition, whether or not calcium phosphate particles are sintered may be determined by the crystalline degree of the corresponding particles. The crystalline degree of calcium phosphate particles maybe measured by an X-ray diffraction method (XRD). As narrower the half maximum full-width of the peak representing each of crystal faces is, the crystallinity may be high. Sintered hydroxyapatite particles indicate high crystalline hydroxyapatite particles having 0.8 or less (more preferably, 0.5 or less) of the half maximum full-width at d = 2.814.

Hydroxyapatite particles are sintered, thereby becoming more strong and stable particles. Therefore, sintered hydroxyapatite particles are smoothly dissolved, and also, exhibits desired acid neutralizing capacity in the neutralization reaction with acidic components. At the same time, even when the particles are dissolved by the acidic components, the particles do not easily collapse, and thus, it is possible to exhibit acid neutralizing capacity over a longer period of time.

### (Particle diameter)

The particle diameter of hydroxyapatite particles used as the biodegradable material according to the present embodiment is 10 nm or more and 500 nm or less. When the biodegradable material according to the present embodiment is applied in vivo, some calcium phosphate particles are fallen off from a polyester base material according to the degradation of the polyester base material, and then, released in vivo. Here, when the particle diameter of calcium phosphate particles is less than 10 nm, there is a possibility that the calcium phosphate particles released in vivo are easily penetrated into the gap between vascular endothelial cells in vivo (in general, there is known to be 15 to 20 nm) due to the calcium phosphate particles having very small size, and then, may be diffused. Meanwhile, the calcium phosphate particles do not easily diffuse through the gap between vascular endothelial cells by making the particle diameter of calcium phosphate particles to be 10 nm or more (the particle diameter which is close to the size of the gap present between vascular endothelial cells or is larger than the size thereof) (in other words, the calcium phosphate particles easily stay in the area with the acidic components generated by degrading the polyester base material) . For this reason, along with the calcium phosphate particles present in the polyester base material (or the surface thereof), the calcium phosphate particles released from the polyester base material are also possible to be contributed as an acid neutralizing component. On the other hand, in a case where the particle diameter of the calcium phosphate particles is more than 1000 nm, when the calcium phosphate particles are dispersed in the polyester base material, it may cause the defect in the corresponding polyester base material, and thus, the mechanical strength of the biodegradable material may be significantly decreased. In addition, in a case where the particle diameter of calcium phosphate particle is inordinately large, the calcium phosphate particles easily fall off from the polyester base material, and thus, the neutralizing capacity for an acidic component generated by degrading the polyester base material is reduced (it is difficult to maintain the pH stability of the whole biodegradable material) . In order to sufficiently exhibit these functions of the calcium phosphate particles, and also, further increase the effect on preventing the decrease in the mechanical strength of a degradable material, the particle diameter of calcium phosphate particle is 10 nm or more and 500 nm or less.

In addition, it is considered that in a case where the particle diameter of calcium phosphate particles is large (for example, in a case where the particle diameter thereof is 1000 nm or more), when a polymer is degraded, all the particles easily fall off in a particle shape as it is from the polyester base material before being reacted by the neutralization of acid (there are many particles that maybe fallen off from the polyester base material before being reacted by the neutralization of acid) . In addition, it is considered that when the particles having large particle diameter are fallen off from the polyester base material, the surface morphology (for example, the present amount of cracks or holes) may be largely changed by such as the holes formed in the corresponding fallen sites. In a case where the particle diameter of calcium phosphate particles is large as described above, when a polymer is degraded, it is expected that the morphology of biodegradable material (the weight of biodegradable material, or especially, the surface morphology of biodegradable material, and the like) easily changes, and thus, turbulent flow easily generates in the bloodstream of the places contacted with the corresponding biodegradable material (the bloodstream is easily disturbed).

In addition, the particle diameter of calcium phosphate particles used for the biodegradable material according to the present embodiment indicates the value obtained by the following method. In the SEM image obtained by photographing calcium phosphate particles, two segments of a line, in which the both ends thereof are located on the outer periphery of the particle, are drawn on the particle. At this time, the length of the segment of a line is allowed to be the largest. In addition, at the middle point of the corresponding segment of a line, another segment of a line is drawn to be lied at right angles to each other. Among two segments of a line drawn as described above, the length of shorter side of the line segments is defined as a short diameter and the length of longer side of the line segments is definedas along diameter. Inaddition, the particle diameter is obtained by obtaining the average value of the corresponding long diameters in 150 particles, in which the particles having large long diameters are sequentially taken in order.

### (Preparing method)

As the calcium phosphate particles according to the present embodiment, the calcium phosphate particles prepared by the general method of preparing calcium phosphate particles may be used. A solution method (wet method), dry method, heated water method, or the like may be used, and especially, when they are industrially produced in large quantities, the solution method (wet method) is used. The solution method (wet method) is a synthesizing method by reacting a calcium ion and a phosphate ion in a neutral or alkaline aqueous solution, and there may be the method by a neutralization reaction or the reaction of a calcium salt and a phosphate salt. In addition, it is possible to use the particles having larger particle diameter than the aggregate of particles, for example, by the sintering of primary particles, or to use the more dense particles. In addition, for example, various hydroxyapatite particles are available in the market such as micro-SHAp (IHM-100P000, Sofsera Corp.), and thus, the particles having various shapes and properties and prepared by various preparing methods may be obtained.

### <Other components>

In addition, the biodegradable material according to the present embodiment may also include antibiotics, anticancer agents, immunosuppressive drugs, cell proliferation inhibitors, antithrombotic drugs, antiplatelet agents, anti-inflammatory, calcium antagonist, antiallergic drugs, antihyperlipidemic drugs, retinoid, flavonoid, carotenoid, lipid, protein, cytokine, vitamins, saccharides, materials of biological origin, inorganic salts, and the like, as other components.

### <<Composition>>

### <Blending amount>

The total content of polyester and calcium phosphate particles is preferably 1 to 100% by mass, more preferably 10 to 100% by mass, and still more preferably 50 to 100% by mass with respect to the mass of the biodegradable material according to the present embodiment.

### <Blending ratio>

The blending ratio of calcium phosphate particles and polyester (calcium phosphate particles : polyester) is preferably 0.01 : 99.99 to 70 : 30, more preferably 0.05 : 99.95 to 60 : 40, and still more preferably 0.1 : 99.9 to 50 : 50 as the mass ratio. By making the blending ratio of polyester and calcium phosphate particles to be in these ranges, it is expected that while maintaining the mechanical strength, the neutralizing effect of acid derived from degradation products may be exhibited.

### <<Physical properties of biodegradable material>>

Next, the respective physical properties of the biodegradable material according to the present embodiment will be described.

### <Mechanical strength>

The biodegradable material according to the present embodiment has the mechanical strength in the level in that a shape retaining property and surface morphology retaining property can be maintained for at least several weeks.

### <pH stability>

The pH stability may be measured by immersing the biodegradable material to be measured as a subject in normal saline solution, maintaining the material at a proper temperature for a proper time, and then, investigating the pH value of the corresponding aqueous solution. In the normal saline solution immersed with the polyester base material, the degradation of polyester base material (releasing acidic components) is performed, thereby changing the pH to be decreased. For the corresponding test, as the pH value measured is close to 7, the pH stability of the biodegradable material is high, and even when the biodegradable material is applied in vivo, it is possible to suppress inflammatory. The biodegradable material according to the present embodiment is composed so that acid neutralizing capacity by calcium phosphate particles is easily exhibited, and thus, the pH stability is high.

### <<Method of preparing biodegradable material>>

As described above, the structure and physical properties of the biodegradable material according to the present embodiment are described, and subsequently, a method of preparing the biodegradable material having the above-described structure and physical properties will be described.

### <Raw materials>

For the raw materials in the method of preparing the biodegradable material according to the present embodiment, the type or preparing method of calcium phosphate particles, type of polyester of a polyester base material, and the blending amount of polyester and calcium phosphate particles are described above, and thus, the detailed descriptions thereof will not be provided hereinafter.

### <Preparing process>

A biodegradable material is prepared by mixing polyester and calcium phosphate particles blended in the blending amount as described above . In addition, any mixing methods may be used as the corresponding mixing method, but for example, there may be the method in which the calcium phosphate particles are mixed by dissolving a polymer in various solvents, or the calcium phosphate particles are mixed by heat-melting a polymer. Next, the corresponding mixture is formed in the shape adjusted for the desired use. As such a forming method, the conventional methods (for example, an injection formation, extrusion formation, blow formation, and the like) may be properly used.

### <<Use of biodegradable material>>

The biodegradable material according to the present embodiment has high pH stability and sufficient mechanical strength, and can be used for various uses. For example, it may be used as a biomaterial, and especially, it may be preferably used as a biomaterial for implant (for example, a stent, and the like).

### <<Examples>>

Hereinafter, the present invention will be described in detail with reference to Examples. In addition, the present invention is not limited to the corresponding Examples.

### <Preparing Example>

### (Preparing of SHAp)

### (Preparing of SHAp/PLGA film)

PLGA (manufactured by Sigma-Aldrich Co. LLC., RG 752 H/lactic acid : glycolic acid = 75 : 25) and SHAp (MHS-004P000, manufactured by SofSera Corp. , sintered hydroxyapatite, 43 nm of average particle diameter) were mixed in the mass ratios (SHAp/PLGA) of 0/100, 1/99, 5/95, 10/90, and 30/70, and then, the corresponding mixtures were formed in the sample (about 400 mg) in a sheet shape.

### <Test method>

The above samples were soaked into the sample tube filled with phosphate normal saline (PBS), the cap was covered thereon, and then, the incubation was performed at 37°C. Since then, the samples were taken out at the different incubation times {0 day (before incubation), for one week, for three weeks} (each of them, n = 7), and dried. Since then, the masses of the samples were measured. Next, the weight change, surface morphology (observation by SEM), and the change of weight average molecular weight were evaluated. In addition, for the comparison, the tensile strength for one without SHAp and the molecular weight of the sample before the incubation in PBS were measured.

### <Results>

### (Weight change)

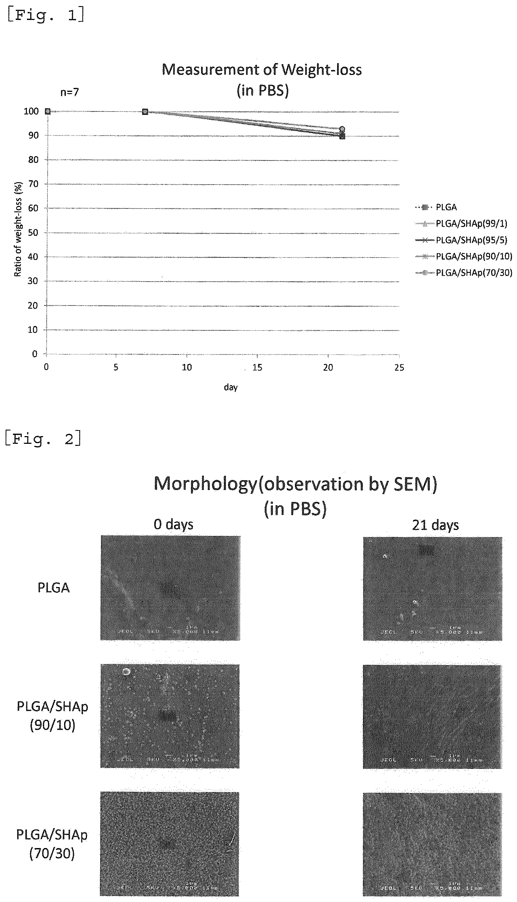

Fig. 1 is a graph illustrating the changes with time of the weights of the films according to Examples and Comparative Examples. From these results, it could be confirmed that all the samples exhibited the same weight decrease trends regardless whether or not SHAp is included. In addition, from the corresponding results, it could be confirmed that since the weight decrease was about 10% even after 21 days, the "form" of the material itself was hardly changed. In other words, it could be confirmed that the surface was not rough by the degradation, and the turbulent flow of bloodstream caused by the degradation of a polymer was not easily generated.

### (Surface morphology)

Fig. 2 is the SEM photographs of the surface morphologies of the films according to Examples and Comparative Examples. As could be confirmed from the corresponding photographs, there were no difference whether or not SHAp is included, and also, even after 21 days, the surface morphology was hardly changed. From this result, it could be determined that even in the system including many SHAp, there were no large cracks or holes on the surface, and after 21 days, even though the degradation was performed, the surface morphology was not largely changed.

### (Molecular weight change)

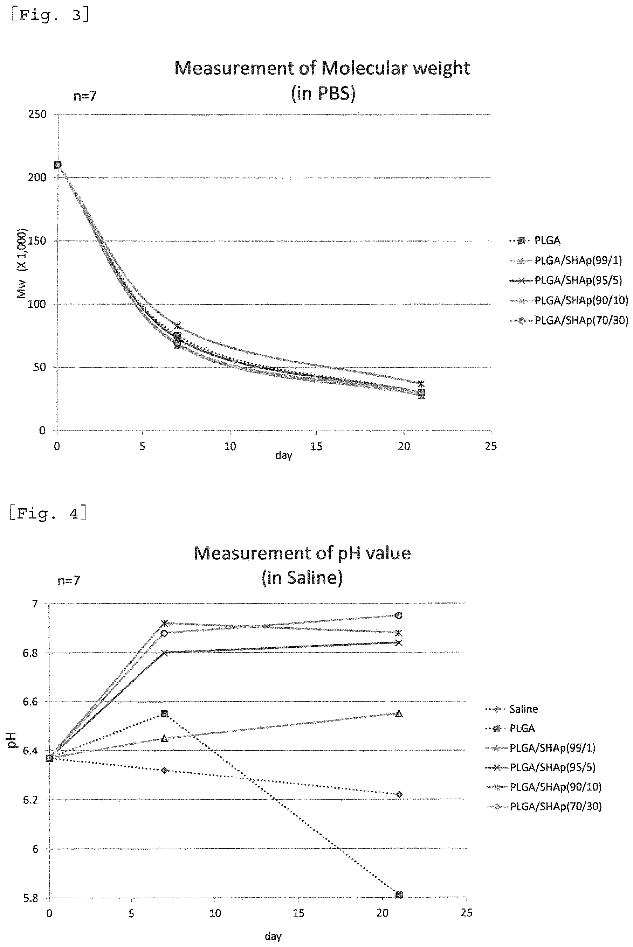

Fig. 3 is a graph illustrating the results of measuring the weight average molecular weights of PLGA films prepared by immersing respective samples according to Examples and Comparative Examples in phosphate buffered normal saline (PBS), leaving the samples as it is at 37°C, and then being collected. In Fig. 3, the vertical axis indicates the molecular weight (the number marked is one in 1000 of the real value) and the horizontal axis indicates the elapsed times. As could be confirmed from Fig. 3, at the start time, the molecular weight was about 210, 000, but on day 7, the degradation was promoted by about 1/3 of the original value. Meanwhile, at a stage after 7 days, there was no difference whether or not SHAp is included. In other words, it was confirmed whether or not SHAp is included did not largely affect the degradation rate of a polymer (the same trend even on day 21). In addition, in the different test, it was confirmed that when the particle diameter was large, since the fallings of particles were generated and the contact area between the material and medium (water in vivo) was increased, the hydrolysis was promoted, and thus the decrease in the molecular weight was accelerated, thereby shortening the time that can maintain the sufficient mechanical strength.

### (PH change)

Fig. 4 is a graph illustrating the result of measuring pH of the solutions prepared by immersing respective samples according to Examples and Comparative Examples in normal saline and leaving the samples as it is at 37°C. At the start point, the pH of solutionwas 6 . 37. In addition, as the value of vertical axis was decreased, the pH was inclined to acidity, and on the contrary, as the value was increased, when it was the neutral pH (7) or more, the pH was inclined to alkalinity. After 7 days, the control group without immersing in normal saline (Saline; a broken line) was 6.32 that was almost steady (slightly, the pH was changed to be acidity over time, but it was influenced by the difference of dissolved amount of carbon dioxide in the air, that is, error range). Inaddition, the control group (PLGA; a broken line), in which the PLGA sheet without SHAp was immersed, was changed to be slightly neutral pH, that is, 6.55. On the other hand, all the groups, in which the PLGA sheet with SHAp was immersed, were changed to be neutral pH. Especially, for Example having 5% by weight or more of SHAp-containing amount, the pH was largely changed to be neutral pH. In addition, after 21 days, the control group without immersing in normal saline was slightly inclined to be acidity, but the pH was almost steady. In addition, the control group, in which the PLGA sheet without SHAp was immersed, was significantly inclined to be acidity. Meanwhile, for all the systems, in which the PLGA sheet with SHAp was immersed, the acidity or alkalinity was clearly changed to be neutral pH as compared with the pH at the start point. As described above, when comparing whether or not SHAp is included, it could be found that there were clear differences as compared with the system without SHAp.

In addition, for the comparison, the particles having the particle diameter of 1 to 5 µm were verified. However, the particles were precipitated during removing a solvent, and the uniformity in the materials could not be maintained. Therefore, it was determined that there were practical problems. In addition, the particles without sintering, as compared with the particles sintered, were less efficient in that the solubility was slightly fast, and thus, the adaptable time was shortened. However, it was confirmed that there were no practical problems. In addition, α-TCP that was not hydroxyapatite, as compared with hydroxyapatite, were less efficient in that it was reacted with water and was first changed to be calcium-deficient hydroxyapatite, and then, the reaction rate was decreased. However, it was confirmed that there were no practical problems.

### <Results>

From the above data, it was determined that the problems, in which the degradation products of biodegradable polymer exhibited acidity conventionally, thereby causing inflammation, could be relieved by SHAp.

## Claims

1. A biodegradable material being a biodegradable material including polyester, which further comprises calcium phosphate particles having an average particle diameter of 10 to 500 nm, wherein the calcium phosphate particles are a sintered body, the calcium phosphate is hydroxyapatite and the hydroxyapatite particles are included in the polyester.

2. The biodegradable material according to claim 1, wherein a content of the calcium phosphate particles is 0.1 to 50% by mass with respect to the total mass of the material.

3. The biodegradable material according to claim 1 or 2, the biodegradable material being for a biomaterial for implants.

4. The biodegradable material according to claim 3, wherein the implants are stents.

5. Use of the biodegradable material according to claim 1 or 2 for a biomaterial for implants.

6. Use of the biodegradable material according to claim 5, wherein the implants are stents.

7. Implants having the biodegradable material according to claim 1 or 2.

8. Implants according to claim 7, wherein the implants are stents.

## Patentansprüche

1. Biologisch abbaubares Material, das ein biologisch abbaubares Material ist, das Polyester enthält, das ferner Calciumphosphatteilchen mit einem mittleren Teilchendurchmesser von 10 bis 500 nm umfasst, wobei die Calciumphosphatteilchen Sinterkörper sind, das Calciumphosphat Hydroxylapatit ist und die Hydroxylapatitteilchen im Polyester eingeschlossen sind.

2. Biologisch abbaubares Material gemäß Anspruch 1, wobei der Gehalt der Calciumphosphatteilchen 0,1 bis 50 Massen-% beträgt, bezogen auf die Gesamtmasse des Materials.

3. Biologisch abbaubares Material gemäß Anspruch 1 oder 2, wobei das biologisch abbaubare Material für ein Biomaterial für Implantate ist.

4. Biologisch abbaubares Material gemäß Anspruch 3, wobei die Implantate Stents sind.

5. Verwendung des biologisch abbaubaren Materials gemäß Anspruch 1 oder 2 für ein Biomaterial für Implantate.

6. Verwendung des biologisch abbaubaren Materials gemäß Anspruch 5, wobei die Implantate Stents sind.

7. Implantate mit dem biologisch abbaubaren Material gemäß Anspruch 1 oder 2.

8. Implantate gemäß Anspruch 7, wobei die Implantate Stents sind.

## Revendications

1. Matériau biodégradable étant un matériau biodégradable comportant du polyester, qui comprend en outre des particules de phosphate de calcium ayant un diamètre de particule moyen de 10 à 500 nm, dans lequel les particules de phosphate de calcium sont un corps fritté, le phosphate de calcium est l'hydroxyapatite et les particules d'hydroxyapatite sont incluses dans le polyester.

2. Le matériau biodégradable selon la revendication 1, dans lequel une teneur des particules de phosphate de calcium est de 0,1 à 50% en masse par rapport à la masse totale du matériau.

3. Le matériau biodégradable selon la revendication 1 ou 2, le matériau biodégradable étant pour un biomatériau pour des implants.

4. Le matériau biodégradable selon la revendication 3, dans lequel les implants sont des endoprothèses.

5. Utilisation du matériau biodégradable selon la revendication 1 ou 2 pour un biomatériau pour des implants.

6. Utilisation du matériau biodégradable selon la revendication 5, dans laquelle les implants sont des endoprothèses.

7. Implants ayant le matériau biodégradable selon la revendication 1 ou 2.

8. Implants selon la revendication 7, dans lesquels les implants sont des endoprothèses.
